# EUROPEAN PATENT APPLICATION

(11) **EP 3 622 959 A1**
(43) Date of publication of application: **18.03.2020**
(21) Application number: 18196162.4
(22) Date of filing: 24.09.2018
(51) Int. Cl.: A61K 31/724, A61P 43/00, A01N 1/02, A61K 35/00

(54) **CYCLODEXTRIN FOR PROTECTING CELLS UPON CRYOPRESERVATION**

(30) Priority: 17.09.2018 US 201862732208 P
(71) Applicant: Roquette America, Inc., Geneva, IL 60134 (US)
(72) Inventor: YASHCHUK, Sofiya, Geneva, IL 60134 (US); FERGUSON, Peter, 62136 Lestrem (FR)
(74) Representative: Plasseraud IP

(57) **Abstract**

The present invention relates to the use of cyclodextrin compounds as cell protecting agents for therapeutic cells. The invention further relates to therapeutic cell compositions comprising such cyclodextrin compounds.

## Description

The present invention relates to the use of cyclodextrin compounds as cell protecting agents for therapeutic cells. The invention further relates to therapeutic cell compositions comprising such cyclodextrin compounds.

### Context of the invention

Average global life expectancy has accelerated by 5 years at the fastest growth rate since 1950 between 2000 and 2015, and now exceeds 71 years. However, with this increase in lifespan comes a growing incidence of chronic human diseases prevalent in aging, such as cancer, cardiovascular and neurodegenerative diseases. As such, sustained progress in extending life expectancy and quality of life requires the continued development of novel therapies and approaches that address chronic human conditions prevalent in an aging population.

A promising development in the treatment of chronic diseases is the use of living cells as 'smart drugs' that can effectively target specific tissues to rescue organ function or eradicate tumors. In the case of cancer, cell therapies have proven effective in patients who have exhausted other lines of standard care treatment, with reported results including complete remission and extension of median survival time. As living drugs, cell therapies need to be viable and functional in order to be effective, and methods for preserving small molecules and monoclonal antibodies are not similarly effective for cell-based products.

Rather, they require cellular support during all phases of the product lifecycle, from source material acquisition to final delivery and patient administration. Optimized strategies to maintain the viable recovery and efficacy of cell therapies during all stages of collection, manufacturing and delivery are essential to realize the potential of these promising medical advances.

Cell therapies originate from biological sources, such as tissue biopsies, blood and bone marrow. This biological seed material is then manipulated in a laboratory or manufacturing facility to develop the cell therapy product. Whether autologous or allogeneic, the development and eventual introduction of the therapeutic dose into a patient involves rounds of transportation between the collection site and the laboratory or cell manufacturing facility, as well as storage periods of varying durations for logistic arrangements and quality control checks. Unlike pharmaceutical or small molecule agents, cells and tissues have unique requirements in order to remain viable and functional while outside the body or culture conditions. Therefore, an essential component of the cell manufacturing lifecycle is to preserve cells during handling and storage. More so, improper preservation of biologic specimens at any step in the process may negatively impact all subsequent manufacturing steps, and may adversely impact the final cell therapy product.

As soon as biological specimens are removed from the body and normothermic conditions, deleterious environmental stresses begin to degrade the source material. From a clinical efficacy standpoint, these environmental stresses are compounded at each step of the cell product lifecycle, introducing sample variability and the potential for impaired therapeutic function, which may even lead to clinical inefficacy and termination of a potentially effective treatment. From a financial standpoint, loss of cell yield, viability and function adds significant cost to cell therapies by increasing the potential for repeat sampling or additional processing steps to expand cell numbers or resuscitate function. Biopreservation refers to the processes required to maintain the health and function of biologics outside the body, as well as suppress the degradation of these biological materials to ensure a return to function post-preservation. By reducing the stresses experienced by cells and tissues *ex vivo,* optimized biopreservation processes can extend the time that cells and tissues remain outside of normothermic conditions. For commercial cell therapy products, effective biopreservation protocols provide flexibility in manufacturing and shipping, facilitate effective process development, and reduce manufacturing costs. An ideal biopreservation protocol would maintain biological function throughout the product lifecycle, providing 'vein-to-vein'/'needle-to-needle' support from the time the sample is collected from the donor to the time it is administered to the recipient.

It results therefrom that there is a high need for improved methods of biopreservation.

### Objective of the invention

It was thus an object of the present invention to provide solution for the biopreservation of therapeutic cells.

### Presentation of the invention

The Applicant found that cyclodextrin compounds, in particular beta-cyclodextrin compounds, are efficient in biopreserving therapeutic cells.

Cyclodextrins are cyclic oligosaccharides which come from the enzymatic degradation of starch. The three most common natural cyclodextrins are made up of 6, 7 or 8 α-D-anhydroglucose units in chair configuration, linked to one another by α-1,4 bonds. They are more commonly referred to as α, β, or γ cyclodextrin, respectively. Their three-dimensional structure appears in the form of a truncated cone on the outside of which are the hydroxyl groups representing the highly hydrophilic part of cyclodextrins. The interior of the cone or the cavity of cyclodextrins is made up of the hydrogen atoms borne by the C₃ and C₅ carbons and also of the oxygen atoms which participate in the glycosidic bond, thus conferring on them a nonpolar nature.

Cyclodextrins having a hydrophilic external part and a hydrophobic cavity are generally used for their ability to encapsulate hydrophobic compounds and, therefore, for their ability to solubilize and stabilize hydrophobic compound in solution.

With a view to improving the aqueous solubility of natural cyclodextrins, many derivatives have been synthesized by grafting various groups onto the hydroxyl functions. The anhydroglucose units of cyclodextrins in fact each comprise 3 reactive hydroxyl groups, which are borne by the C₂, C₃ and C₆ carbons. As examples of derivatives, mention may be made of alkylated cyclodextrins like hydroxypropyl-beta-cyclodextrins (HPβCD), methyl-beta-cyclodextrins (MβCD) and "sulfated" derivatives of beta-cyclodextrin (SBEβCD).

However, the use thereof in biopreservation was never considered.

### Summary of the invention

The invention first relates to a method of biopreservation of therapeutic cells, comprising the use of a cyclodextrin compound.

The invention also relates to a therapeutic cell composition comprising such cyclodextrin compound.

### Detailed description of the invention

The invention first relates to a method of biopreservation of therapeutic cells, comprising the use of a cyclodextrin compound.

It is commonly understood that the expression « cyclodextrin compound» refers to a mixture of cyclodextrin molecules, further comprising the substances resulting from its manufacturing process. Such cyclodextrin compound might be a substituted cyclodextrin, in particular alkylated cyclodextrin, like for instance methyl-cyclodextrin, hydroxypropyl-cyclodextrin or sulfobutylether-cyclodextrin. Contrary to chemical substances with well-defined structure, alkylated cyclodextrins generally are a mixture of alkylated cyclodextrin molecules having different substitution profiles, and which are thus structurally different.

In a first embodiment, the cyclodextrin compound is unsubstituted cyclodextrin, preferably beta-cyclodextrin.

In another embodiment, the cyclodextrin compound of the invention is a substituted cyclodextrin, in particular alkylated cyclodextrin, preferably selected from hydroxypropyl-cyclodextrin, methyl-cyclodextrin, and sulfobutylether-cyclodextrin.

Preferably, the alkylated-cyclodextrin of the invention has an average molar substitution degree (MS) ranging from 0.10 to 1.50, preferably from 0.20 to 1.40, preferably from 0.30 to 1.30, preferably from 0.40 to 1.20, preferably from 0.50 to 1.10, preferably from 0.55 to 1.00, preferably from 0.58 to 0.99.

It is reminded the "average molar substitution degree (MS)" refers to the average number of alkyl groups per anhydroglucose unit. It should be noted that this MS is different from the average molecular substitution degree (DS), which refers to the average number of alkyl groups per cyclodextrin molecule, and which is, as a consequence, function of the number of anhydroglucose units forming the original cyclodextrin. For instance, for alkylated beta-cyclodextrins, this DS is equal to 7 times the MS, as the β-cyclodextrins are made of 7 anhydroglucose units.

In a preferred embodiment, the alkylated cyclodextrin of the invention is hydroxypropyl-cyclodextrin, preferably hydroxypropyl-beta-cyclodextrin (HPβCD).

Preferably, the hydroxypropyl-cyclodextrin of the invention, in particular HPβCD, has an average molar substitution degree (MS) within the range described above. The MS of the hydroxypropyl-cyclodextrin of the invention, in particular of the HPβCD, can classically be determined by the person skilled in the art by proton nuclear magnetic resonance (NMR), preferably according to the USP 41 NF 36 method « Hydroxypropyl Betadex ; Molar substitution ».

Preferably, the hydroxypropyl-cyclodextrin of the invention, in particular HPβCD, has the following substitution profile, as determined by electrospray ionization - Mass spectrometry (ESI-MS):
- signal corresponding to unsubstituted cyclodextrin (HP0) : equal to or lower than 5 %, preferably equal to or lower than 4 %, preferably equal to or lower than 3 %, preferably equal to or lower than 2 %, preferably equal to or lower than 1 %, preferably equal to 0 %; and/or,
- signal corresponding to the hydroxypropyl-cyclodextrin molecules having 1 substitution (HP1): equal to or lower than 5%, preferably equal to or lower than 4 %, preferably equal to or lower than 3 %, preferably equal to or lower than 2 %, preferably equal to or lower than 1 %; and/or,
- signal corresponding to the hydroxypropyl-cyclodextrin molecules having 2 substitutions (HP2): equal to or lower than 5 %, preferably equal to or lower than 4 %, preferably equal to or lower than 3 %, preferably equal to or lower than 2 %; and/or,
- signal corresponding to the hydroxypropyl-cyclodextrin molecules having 3 substitutions (HP3): selected within the range of from 0 to 15 %, preferably of from 0 to 10 %, preferably of from 0 to 9 %, preferably of from 0 to 8 %, preferably of from 0 to 6 %; and/or,
- signal corresponding to the hydroxypropyl-cyclodextrin molecules having 4 substitutions (HP4): selected within the range of from 0 to 25 %, preferably of from 0 to 20 %, preferably of from 0 to 15 %, preferably of from 1 to 13 %; and/or,
- signal corresponding to the hydroxypropyl-cyclodextrin molecules having 5 substitutions (HP5): selected within the range of from 0 to 40 %, preferably of from 0 to 35 %, preferably of from 0 to 30 %, preferably of from 1 to 25 %; and/or
- signal corresponding to the hydroxypropyl-cyclodextrin molecules having 6 substitutions (HP6): selected within the range of from 0 to 50 %, preferably of from 1 to 45 %, preferably of from 3 to 40 %, preferably of from 5 to 35 %, preferably of from 10 to 30 %; and/or
- signal corresponding to the hydroxypropyl-cyclodextrin molecules having 7 substitutions (HP7): selected within the range of from 5 to 50 %, preferably of from 10 to 40 %, preferably of from 15 to 30 %, and/or,
- signal corresponding to the hydroxypropyl-cyclodextrin molecules having 8 substitutions (HP8): selected within the range of from 1 to 50 %, preferably of from 2 to 45 %, preferably of from 3 to 40 %, preferably of from 4 to 35 %, preferably of from 5 to 30 %; and/or,
- signal corresponding to the hydroxypropyl-cyclodextrin molecules having 9 substitutions (HP9): selected within the range of from 0 to 30 %, preferably of from 1 to 25 %; and/or,
- signal corresponding to the hydroxypropyl-cyclodextrin molecules having 10 substitutions (HP10): selected within the range of from 0 to 20 %, preferably of from 0 to 15 %, preferably of from 0 to 10 %; and/or,
- signal corresponding to the hydroxypropyl-cyclodextrin molecules having 11 substitutions (HP11): lower than or equal to 10 %, preferably lower than or equal to 8 %, preferably equal to or lower than 5 %, preferably equal to or lower than 3 %; and/or
- signal corresponding to the hydroxypropyl-cyclodextrin molecules having 12 substitutions (HP12): lower than or equal to 5 %, preferably equal to or lower than 4 %, preferably equal to or lower than 3 %, preferably equal to or lower than 2 %, preferably equal to or lower than 1 %, and/or
- signal corresponding to the hydroxypropyl-cyclodextrin molecules having 13 or more substitutions (HP≥13): equal to or lower than 5%, preferably equal to or lower than 4 %, preferably equal to or lower than 3 %, preferably equal to or lower than 2 %, preferably equal to or lower than 1 %, preferably equal to 0 %;
this percentages being expressed with respect of the sum of the signals obtained for each substitution for which the signal was higher than the background.

It is understood that "signal" refers to the area under the curve of the ion(s) corresponding to the substitutions degree(s) of interest.

In the present invention, the substitution profile is determined by ESI-MS, preferably by calculating the average of the measurements performed in triplicate. For determining this substitution profile, it is in particular possible to proceed according to the following method:
- A solution hydroxypropyl-cyclodextrin is prepared at 1 g.L⁻¹ (w/v) in methanol:
   water (50/50, v/v) with 1 mM sodium acetate. Infusion is performed for 1 min at 10 µL/min and data are recorded. In between two following injections, 500 µL of methanol: water (50/50, v/v) are injected to wash the ion source.
- ESI parameters: Spray voltage: 5 kV; Sheath gas: 9; Auxiliary gas: 2; Sweep gas: 0; Spray voltage: 5 kV; Capillary voltage: 23 V; Capillary temperature: 275°C; Tube lens: 80 V
- MS parameters: Full scan; Scan ranges: 50-2000 m/z; Mass range: normal; Scan rate: enhanced; Data acquisition time: 1 min.
- For each substituted hydroxypropyl-cyclodextrin species (named as HPX, X being the number of hydroxypropyl substitutions), the corresponding ion current (XIC) is integrated and compared to the sum of all HPX ion currents. As the sodium adduct is the most intense hydroxypropyl-cyclodextrin ion (almost 100 times superior to [M+H]⁺ or [M+NH4]⁺), its peak area is integrated to estimate the proportion of the corresponding HPX molecule.

In a particular preferred embodiment, the hydroxypropyl-cyclodextrin of the invention, in particular HPβCD, has:
- a MS ranging from 0.50 to 0.75, preferably from 0.55 to 0.70, preferably from 0.58 to 0.68; and/or,
- the following substitution profile, as determined by electrospray ionization - Mass spectrometry (ESI-MS):
   - HP0: equal to or lower than 5 %, preferably equal to or lower than 4 %, preferably equal to or lower than 3 %, preferably equal to or lower than 2 %, preferably equal to or lower than 1 %, preferably equal to 0 %; and/or,
   - HP1: equal to or lower than 5 %, preferably equal to or lower than 4 %, preferably equal to or lower than 3 %, preferably equal to or lower than 2 %, preferably equal to or lower than 1 %; and/or,
   - HP2: equal to or lower than 5 %, preferably equal to or lower than 4 %, preferably equal to or lower than 3 %, preferably equal to or lower than 2 %; and/or,
   - HP3: selected within the range of from 0 to 15 %, preferably of from 1 to 10 %, preferably of from 1 to 8 %, preferably of from 2 to 6 %; and/or,
   - HP4: selected within the range of from 0 to 25 %, preferably of from 2 to 20 %, preferably of from 4 to 15 %, preferably of from 7 to 13 %; and/or,
   - HP5: selected within the range of from 1 to 40 %, preferably of from 5 to 35 %, preferably of from 10 to 30 %, preferably of from 15 to 25 %; and/or
   - HP6: selected within the range of from 5 to 50 %, preferably of from 10 to 40 %, preferably from 15 to 35 %, preferably of from 20 to 30 %; and/or
   - HP7: selected within the range of from 5 to 50 %, 10 to 40 %, preferably of from 15 to 35 %, preferably of from 20 to 30 %; and/or,
   - HP8: selected within the range of from 2 to 30 %, preferably of from 2 to 25 %, preferably of from 5 to 20 %, preferably of from 8 to 15 %; and/or,
   - HP9: selected within the range of from 1 to 10 %, preferably of from 1 to 8 %, preferably of from 2 to 5 %; and/or,
   - HP10: equal to or lower than 5 %, preferably equal to or lower than 4 %, preferably equal to or lower than 3 %, preferably equal to or lower than 2 %, preferably equal to or lower than 1 %; and/or,
   - HP≥11: equal to or lower than 5 %, preferably equal to or lower than 4 %, preferably equal to or lower than 3 %, preferably equal to or lower than 2 %, preferably equal to or lower than 1 %, preferably equal to 0 %;
   these percentages being expressed with respect to the sum of the signals obtained for each substitution for which the signal was higher than the one of the background.

In another particular preferred embodiment, the hydroxypropyl-cyclodextrin of the invention, in particular HPβCD, has:
- a MS ranging from 0.70 to 1.20, preferably from 0.75 to 1.10, preferably from 0.80 to 1.00, in particular from 0.81 to 0.99; and/or,
- the following substitution profile, as determined by electrospray ionization - Mass spectrometry (ESI-MS):
   - HP≤2 : equal to or lower than 5 %, preferably equal to or lower than 4 %, preferably equal to or lower than 3 %, preferably equal to or lower than 2 %, preferably equal to or lower than 1 %, preferably equal to 0 %; and/or,
   - HP3: equal to or lower than 5 %, preferably equal to or lower than 4 %, preferably equal to or lower than 3 %, preferably equal to or lower than 2 %, preferably equal to or lower than 1 %; and/or,
   - HP4: selected within the range of from 0 to 5 %, preferably of from 0 to 4 %, preferably of from 1 to 3 %; and/or,
   - HP5: selected within the range of from 1 to 15 %, preferably of from 2 to 10 %, preferably of from 3 to 8 %; and/or
   - HP6: selected within the range of from 2 to 30 %, preferably of from 5 to 25 %, preferably from 7 to 20 %, preferably of from 10 to 20 %; and/or
   - HP7: selected within the range of from 5 to 45 %, 10 to 40 %, preferably of from 15 to 35 %, preferably of from 20 to 30 %; and/or,
   - HP8: selected within the range of from 5 to 50 %, preferably of from 10 to 40 %, preferably of from 15 to 35 %, preferably of from 20 to 30 %; and/or,
   - HP9: selected within the range of from 3 to 40 %, preferably of from 5 to 35 %, preferably of from 10 to 30 %, preferably from 11 to 25 %, preferably from 15 to 25 %; and/or,
   - HP10: selected within the range of from 1 to 20 %, preferably of from 3 to 15 %, preferably of from 5 to 11 %; and/or,
   - HP11: selected within the range of from 0 to 10 %, preferably of from 1 to 5 %; and/or,
   - HP12: equal to or lower than 5 %, preferably equal to or lower than 4 %, preferably equal to or lower than 3 %, preferably equal to or lower than 2 %, preferably equal to or lower than 1 %; and/or,
   - HP≥13: equal to or lower than 5 %, preferably equal to or lower than 4 %, preferably equal to or lower than 3 %, preferably equal to or lower than 2 %, preferably equal to or lower than 1 %, preferably equal to 0 %;
these percentages being expressed with respect to the sum of the signals obtained for each substitution for which the signal was higher than the one of the background.

In another particular preferred embodiment, the hydroxypropyl-cyclodextrin of the invention, in particular HPβCD, has:
- a MS ranging from 0.50 to 0.80, preferably from 0.55 to 0.75, preferably from 0.58 to 0.71; and/or,
- the following substitution profile, as determined by electrospray ionization - Mass spectrometry (ESI-MS):
   - HP0 : equal to or lower than 5 %, preferably equal to or lower than 4 %, preferably equal to or lower than 3 %, preferably equal to or lower than 2 %, preferably equal to or lower than 1 %, preferably equal to 0 %; and/or,
   - HP1: equal to or lower than 5 %, preferably equal to or lower than 4 %, preferably equal to or lower than 3 %, preferably equal to or lower than 2 %, preferably equal to or lower than 1 %; and/or,
   - HP2: equal to or lower than 5 %, preferably equal to or lower than 4 %, preferably equal to or lower than 3 %, preferably equal to or lower than 2 %; and/or,
   - HP3: selected within the range of from 1 to 10 %, preferably of from 2 to 8 %, preferably of from 3 to 7 %; and/or,
   - HP4: selected within the range of from 2 to 25 %, preferably of from 5 to 20 %, preferably of from 6 to 17 %, preferably of from 8 to 15 %; and/or,
   - HP5: selected within the range of from 5 to 40 %, preferably of from 10 to 30 %, preferably of from 15 to 25 %, preferably of from 17 to 25 %; and/or,
   - HP6: selected within the range of from 5 to 45 %, preferably of from 10 to 40 %, preferably of from 15 to 35 %, preferably of from 20 to 30 %, preferably of from 24 to 28 %; and/or,
   - HP7: selected within the range of from 5 to 40 %, preferably of from 10 to 30 %, preferably of from 15 to 25 %; and/or,
   - HP8: selected within the range of from 2 to 20 %, preferably of from 5 to 20 %, preferably of from 5 to 15 %; and/or,
   - HP9: selected within the range of from 1 to 10 %, preferably of from 2 to 8 %, preferably of from 2 to 6 %, and/or,
   - HP10: equal to or lower than 5 %, preferably equal to or lower than 4 %, preferably equal to or lower than 3 %, preferably equal to or lower than 2 %, preferably equal to or lower than 1 %; and/or,
   - HP≥11: equal to or lower than 5 %, preferably equal to or lower than 4 %, preferably equal to or lower than 3 %, preferably equal to or lower than 2 %, preferably equal to or lower than 1 %, preferably equal to 0 %;
   these percentages being expressed with respect to the sum of the signals obtained for each substitution for which the signal was higher than the one of the background.

Hydroxypropyl-cyclodextrins useful to the invention, in particular HPβCD, are commercially available. For instance, the HPβCD marketed under the names KLEPTOSE® HP and KLEPTOSE® HPB (ROQUETTE) are particularly suitable. Another HPβCD useful to the invention is described in the French patent application No. 1855952, the content of which is incorporated by reference.

Preferably, the hydroxypropyl-cyclodextrins of the invention, in particular HPβCDs, have a propylene glycol content equal to or lower than 5.00 %, this percentage being expressed by dry weight of propylene glycol relative to the total dry weight of hydroxypropyl-cyclodextrin. This propylene glycol content is preferably equal to or lower than 2.50 %, preferably equal to or lower than 1.00 %, preferably equal to or lower than 0.50 %, preferably equal to or lower than 0.10 %, preferably equal to or lower than 0.05 %. This propylene glycol content can be classically determined by the person skilled in the art, by high performance liquid chromatography (HPLC), preferably according to a method complying with the USP 41 NF 36 method "Hydroxypropyl Betadex monograph; limit of betadex, propylene glycol, and other related substances".

Preferably, the hydroxypropyl-cyclodextrins of the invention, in particular HPβCDs, have a dipropylene glycol content equal to or lower than 1.00 %, this percentage being expressed by dry weight of dipropylene glycol relative to the total dry weight of hydroxypropyl-cyclodextrin. This dipropylene glycol content is preferably equal to or lower than 0.10 %, preferably equal to or lower than 0.05 %, preferably equal to or lower than 0.03 %, for instance within the range of from 0.01 to 0.05 %..

In another preferred embodiment, the alkylated cyclodextrin of the invention is methyl-cyclodextrin, preferably methyl-beta-cyclodextrin (MβCD).

Preferably, the methyl-cyclodextrin of the invention, in particular MβCD, has an average molar substitution degree (MS) selected within the range of from 0.10 to 1.40, preferably of from 0.10 to 1.30, preferably of from 0.20 to 1.20, preferably of from 0.30 to 1.10, preferably of from 0.30 to 1.00, preferably of from 0.50 to 0.90, preferably of from 0.60 to 0.80, preferably of from 0.60 to 0.70.

The MS of the methyl-cyclodextrin of the invention may conventionally be determined by those skilled in the art by proton nuclear magnetic resonance (NMR), or by mass spectrometry (electrospray ionization mass spectrometry (ESI-MS) or matrix-assisted laser desorption/ionization mass spectrometry (MALDI-MS)). While these techniques are well known to those skilled in the art, those skilled in the art may for example refer to the methods described in the reference thesis by Roman Jacquet: "Cyclodextrines hydrophiles: caracterisation et étude de leurs propriétés enantioselective et complexante. Utilisation de la chromatographie en phase liquide et de la spectrometrie de masse" ["Hydrophilic cyclodextrins: characterization and study of their enantioselective and complexing properties. Use of liquid chromatography and mass spectrometry"]. Thesis on the chemistry and physicochemistry of compounds of biological interest. University of Orleans, 2006. Especially available at: http://tel.archives-ouvertes.fr/docs/00/18/55/42/PDF/jacquet.pdf (consulted on 11.27.2013), in particular Chapter 2, Part B (pages 59 to 83).

Preferably, the MS is determined by NMR, according to the following:
- measurements are taken at 25°C, and the calibration is carried out with the D₂O signal. Samples of MCD, and of unsubstituted cyclodextrin, are prepared at a concentration of 5 mg in 0.75 ml of D₂O. The solutions are evaporated to dryness under a nitrogen stream and then reconstituted in 0.75 ml of D₂O. This operation is repeated twice in order to ensure total exchange of the protons of the hydroxyl functions. The MS is calculated from the difference in integration between the spectrum of the unsubstituted cyclodextrin and that of the methyl cyclodextrin.

The NMR spectrum also makes it possible to calculate the substitution profile.

The methyl cyclodextrin according to the invention may be substituted on the hydroxyl borne by the C2 carbon of the anhydroglucose units, or by the C3 and/or C6 carbons of the anhydroglucose units or by a combination of the C2, C3 and/or C6, preferably C2 and C6, carbons of the anydroglucose units.

This distribution of the methyl groups on the hydroxyls of the anhydroglucose unit of the methyl cyclodextrin may be conventionally determined by those skilled in the art by NMR.

Preferably, at least 50% of the methyl groups of the methyl cyclodextrin of the invention are located at the hydroxyl borne by the C2 carbon of the anhydroglucose unit, for example 50 to 80%, preferentially 60 to 80%, preferentially 65 to 80%, preferentially 70 to 80%, for example 75%.

In parallel, the other methyl groups are generally predominantly located at the hydroxyl borne by the C3 and/or C6 carbon of the anhydroglucose unit.

Advantageously, the methyl cyclodextrin of the invention is methyl-β-cyclodextrin.

In this case, the methyl-p-cyclodextrin preferentially has the following substitution profile:
∘ 0% to 5% of methyl-β-cyclodextrins comprise 2 methyl groups;
∘ 5% to 15% of methyl-β-cyclodextrins comprise 3 methyl groups;
∘ 20% to 25% of methyl-β-cyclodextrins comprise 4 methyl groups;
∘ 25% to 40% of methyl-β-cyclodextrins comprise 5 methyl groups;
∘ 15% to 25% of methyl-β-cyclodextrins comprise 6 methyl groups;
∘ 5% to 15% of methyl-β-cyclodextrins comprise 7 methyl groups;
∘ 0% to 5% of methyl-β-cyclodextrins comprise 8 methyl groups;
these percentages being molar percentages, and the total sum thereof being generally about 100%, although the composition may optionally contain traces of methyl cyclodextrins of different DS, and also traces of residual beta-cyclodextrin, i.e. unsubstituted cyclodextrin.

The substitution profile may be conventionally determined by those skilled in the art, for example by ESI-MS or MALDI-TOF-MS. Although these techniques are well known to those skilled in the art, those skilled in the art may for example refer to the methods described in the abovementioned thesis by Romain Jacquet in chapter 2, part B, points II.3 and II.2 (page 67 to 82) and in appendix II.

Generally and advantageously, the methyl cyclodextrin of the invention comprises less than 20 ppm by dry weight of methylating agent, especially of dimethyl sulfate, preferentially less than 10 ppm, preferentially less than 5 ppm, more preferentially still less than 2 ppm.

The cyclodextrin compounds of the invention advantageously have low reducing sugar content. More specifically, the cyclodextrin compounds of the invention preferably have a reducing sugars content equal to or lower than 5.0 %, this percentage being expressed by dry weight of reducing sugars relative to the total dry weight of the cyclodextrin compound. It is preferably equal to or lower than 1.0 %, preferably equal to or lower than 0.5 %, preferably equal to or lower than 0.3 %, preferably equal to or lower than 0.2 %, preferably equal to or lower than 0.1 %.

This reducing sugar content can be determined by the person skilled in the art for instance according to the well-known "Bertrand method" by precipitation with cuprous oxide in reducing media, filtration on a sintered-glass filter, and weighting of the residue.

The cyclodextrin compounds of the invention preferably have low content of light-absorbing impurities, i.e. low absorbance when they are in solution.

When the cyclodextrin compound in an alkylated cyclodextrin, it preferably has a maximum absorbance, from 230 to 400 nm, equal to or lower than 1.00; said maximum absorbance being determined on a solution comprising 2.50 g (dry) of alkylated cyclodextrin per 100 mL of distilled water, by using a cell having an optical path length of 10 mm. Preferably, this maximum absorbance is equal to or lower than 0.50, preferably equal to or lower than 0.10, preferably equal to or lower than 0.05, for instance within the range of from 0.01 to 0.50.

When the cyclodextrin compound in an unsubstituted cyclodextrin, it preferably has a maximum absorbance from 230 to 350 nm, equal to or lower than 0.10, and/or, a maximum absorbance from 350 to 750 nm, equal to or lower than 0.05. These absorbances of the unsubstituted cyclodextrin can be determined by the person skilled in the art for instance according to the USP 41 NF 36 method "betadex monograph; limit of light-absorbing impurities".

Preferably, the cyclodextrin compounds of the invention have a chloride content equal to or lower than 1000 ppm, preferably equal to or lower than 500 ppm, preferably equal to or lower than 100 ppm, preferably equal to or lower than 50 ppm, preferably lower than 50 ppm; this content being expressed by dry weight of chloride ions, relative to the total dry weight of the cyclodextrin compound. This chloride content can be classically determined by the person skilled in the art by potentiometric titration of a solution of said cyclodextrin compound, by way of a silver nitrate solution of known concentration.

Preferably, the cyclodextrin compounds of the invention have a phosphate content equal to or lower than 1000 ppm, preferably equal to or lower than 500 ppm, preferably equal to or lower than 100 ppm, preferably equal to or lower than 50 ppm, preferably equal to or lower than 10 ppm, preferably equal to or lower than 5 ppm; this content being expressed by dry weight of phosphate, relative to the total dry weight of the cyclodextrin compound.

Preferably, the cyclodextrin compounds of the invention have an alkali metal halide salts content equal to or lower than 1.0%, preferably equal to or lower than 0.5%, preferably equal to or lower than 0.2%, preferably equal to or lower than 0.1%; this percentage being expressed by dry weight of alkali metal halide salts relative to the total dry weight of the cyclodextrin compound.

Preferably, the cyclodextrin compounds of the invention are in a powdery form. In such case, the cyclodextrin compound preferably has loss on drying (or "water content") equal to or lower than 14.0 % by weight, preferably equal to or lower than 12.0 %, preferably equal to or lower than 1.0 %, preferably equal to or lower than 8.0 %, preferably equal to or lower than 5.0 %, for instance within the range of from 2.0 to 5.0 %. This powdery form is advantageous for the transport and storage of the cyclodextrin compound. Preferably, this powdery cyclodextrin compound is in the spray-dried form, i.e. in the form of a product obtained by spray-drying a solution of said cyclodextrin compound.

The cyclodextrin compounds of the invention can also be characterized by its pH, which classically ranges from 5.0 to 8.0. Preferably, when the cyclodextrin compound of the invention is an alkylated cyclodextrin, its pH is within the range of from 5.0 to 7.5, preferably of from 5.0 to 7.0, preferably of from 5.0 to 6.0; this pH being measured at 25°C, on a solution composed of 2 g (dry) of alkylated cyclodextrin, 98 g of distilled water having a resistivity higher than 500 000 ohms.cm, and 0.3 mL of a solution of potassium chloride at 225 g/L. Preferably, when the cyclodextrin compound of the invention is an unsubstituted cyclodextrin, its pH is within the range of from 5.0 to 8.0, as determined according to the USP 41 NF 36 method "betadex monograph; pH".

Preferably, the cyclodextrin compounds of the invention have low conductivity. Preferably, when the cyclodextrin compound of the invention is an alkylated cyclodextrin, its conductivity is equal to or lower than 200 µS/cm, preferably equal to or lower than 100 µS/cm, preferably equal to or lower than 50 µS/cm, preferably equal to or lower than 25 nS/cm, preferably equal to or lower than 10 µS/cm, for instance within the range of from 1 to 10 µS/cm, or even from 2 to 5 µS/cm; this conductivity being calculated from the resistivity measured at 25°C on a 100 mL solution prepared in water having a resistivity greater than 500 000 ohms.cm with a concentration of 10% (dry) of alkylated cyclodextrin. Preferably, when the cyclodextrin compound of the invention is an unsubstituted cyclodextrin, its conductivity is equal to or lower than 200 µS/cm, preferably equal to or lower than 100 µS/cm, preferably equal to or lower than 50 µS/cm, preferably equal to or lower than 25 µS/cm, preferably equal to or lower than 10 µS/cm; this conductivity being measured on a solution composed of 1 g (dry) of beta-cyclodextrin and 99 g of distilled water.

Alkylated cyclodextrins classically include residual cyclodextrin, i.e. unsubstituted cyclodextrin. Preferably, the alkylated cyclodextrins of the invention, have a cyclodextrin content equal to or lower than 5.0 %, preferably equal to or lower than 3.0 %, preferably equal to or lower than 1.0 %, preferably equal to or lower than 0.8 %, preferably equal to or lower than 0.6 %, preferably equal to or lower than 0.5 %, preferably equal to or lower than 0.4 %, preferably equal to or lower than 0.3 %, preferably equal to or lower than 0.2 %, preferably equal to or lower than 0.1 %; this percentage being expressed by dry weight of cyclodextrin relative to the total dry weight of the alkylated cyclodextrin. This residual cyclodextrin content, in particular residual beta-cyclodextrin, can be classically determined by the person skilled in the art by high performance liquid chromatography (HPLC). It can for instance be measured, for HPβCD, according to a method complying with USP 41 NF 36 method « Hydroxypropyl Betadex; limit of betadex, propylene glycol, and other related substances ».

Preferably, the cyclodextrins compound of the invention for which a US monograph is available on September 12^{th} 2018, do comply with the US monograph as in force on September 12^{th} 2018. Preferably, the cyclodextrins compound of the invention for which a European monograph is available on September 12^{th} 2018, do comply with the European monograph as in force on September 12^{th} 2018.

The cyclodextrin compounds as described above are suitable for better biopreservation of the cells, i.e. as cell protecting agents. The invention thus relates to a method of biopreservation of therapeutic cells, comprising the use of a cyclodextrin compound as described above.

"Therapeutic cells" commonly refer to the cells intended for use in veterinary or human cell therapy, preferably human cell therapy.

"Biopreservation" classically refers to the processes aimed to prevent or reduce the ex-*vivo* loss of the therapeutic function(s) of the cells. Biopreservation can be carried out at all steps, from cell harvesting to cell administration, that is: at harvesting the cells, and/or at processing the cells, and/or at storing and/or transporting the cells, and/or at using the cells. "processing the cells" typically refers to the manipulations performed on the harvested cells to develop the cell therapy product (cell culture, cell modification etc.).

Main examples of biopreservation processes are those using low-temperature conditions. In those processes, the cells are placed under low-temperature conditions (hypothermic or cryogenic conditions) in order to reduce its metabolic needs, for instance during storage and transportation. Prior to use, the cold cell preparation is placed again under normothermic conditions (warming) in order to recover its therapeutic function. Despite the metabolic benefits of low-temperature biopreservation, both temperature reduction and return to normothermia exerts unique stresses that can impair the therapeutic function of the cells.

Further or alternatively to the use of low-temperature conditions, optimizing the *ex-vivo* cell media can improve biopreservation. The *ex-vivo* cell media can be the media in which the cells are harvested, and/or processed, and/or stored and/or transported, and/or administrated. In the latter case, biopreservation enables to prevent or reduce the loss of the therapeutic function of the cells when used, in order to improve the shelf life and/or biological function of the therapeutic cells in treated patients.

In the method of the invention, the cyclodextrin compound is put into contact with the therapeutic cells. Preferably, the cyclodextrin compound is placed in the cell media used for harvesting the cells, and/or in the media used for processing the cells, and/or in the media used at storing and/or at transporting the cells, and/or in the cell-containing medicine to be administrated.

Preferably, the method of biopreservation of therapeutic cells of the invention is a method using low-temperature conditions, preferably using hypothermic or cryogenic conditions. In such case, the cyclodextrin compound of the invention is preferably at least put into contact with the cells during the low-temperature treatment, and/or into the cold cell preparation obtained from the low-temperature treatment, and/or into the cold cell preparation to be warmed, and/or during warming, and/or after warming. Preferably, the biopreservation method according to the invention is for preventing or reducing the loss of the therapeutic function of the cells at cooling, and/or at storing and/or at transporting under low-temperature conditions, and/or at warming, for instance at thawing.

In another or complementary embodiment, the method of biopreservation of therapeutic cells is a method for preventing or reducing the loss of the therapeutic function of the cells when administrated. In such case, the cyclodextrin compound of the invention is advantageously co-administrated with the therapeutic cells.

Preferably, the method further comprises the use of amino acids, lipids, in particular cell membrane lipids, vitamin E (tocopherols), and/or cholesterols, preferably concomitantly to the use of the cyclodextrin compound. Preferably, when used in combination with the cyclodextrin compound, these additional substances are complexed with the cyclodextrin compound.

The invention also relates to a therapeutic cell composition comprising a cyclodextrin compound as described above.

Preferably, the therapeutic cell composition further comprises amino acids, lipids, in particular cell membrane lipids, vitamin E (tocopherols), and/or cholesterols. Preferably, when present in said composition, these additional substances are complexed with the cyclodextrin compound.

The therapeutic cell compositions of the invention can be any *ex-vivo* composition comprising the therapeutic cells. It can be for instance the composition obtained at harvesting the cells, the composition in which the cells are processed, the composition in which the therapeutic cells are stored and/or transported, or the cell-containing medicine intended to be administrated.

Preferably, the therapeutic cell composition of the invention is a hypothermic or cryopreserved composition, and/or a cell-containing medicine to be administrated.

Preferably, the therapeutic cells of the invention are CAR T-cells, preferably of human origin.

Preferably, in the methods of biopreservation or therapeutic cell compositions according to the invention, the cyclodextrin compounds enable to reduce or prevent the oxidative stress endured by the cells, especially when the biopreservation process comprises treating the cells under hypothermic or cryogenic conditions.

### Examples

The following products were tested for their ability to promote biopreservation:
- Beta-cyclodextrin (KLEPTOSE® beta-cyclodextrin, ROQUETTE)
- Hydroxypropyl-beta-cyclodextrin with an MS of 0.81-0.99 (KLEPTOSE® HP, ROQUETTE)
- Hydroxypropyl-beta-cyclodextrin with an MS of 0.58-0.68 (KLEPTOSE® HPB, ROQUETTE)
- Methyl-beta-cyclodextrin with an MS of 0.67 as described in US Patent application No 15/767,958 (ROQUETTE).
- Methyl-beta-cyclodextrin with an MS of 0.67 (KLEPTOSE® Crysmeb, ROQUETTE).

These cyclodextrins were found to be efficient as cell protecting agents, for cells intended to be used in cell therapy.

## Claims

1. A method of biopreservation of therapeutic cells comprising the use of a cyclodextrin compound.

2. The method of claim 1, wherein said cyclodextrin compound is unsubstituted cyclodextrin.

3. The method of claim 2, wherein said unsubstituted cyclodextrin is beta-cyclodextrin.

4. The method of claim 1, wherein said cyclodextrin compound is alkylated cyclodextrin.

5. The method of claim 4, wherein said alkyl-cyclodextrin is alkyl-beta-cyclodextrin.

6. The method of any of claims 4 or 5, wherein said alkylated cyclodextrin has an average molar substitution degree (MS) ranging from 0.10 to 1.50.

7. The method of claim 6, wherein said alkylated cyclodextrin has an MS ranging from 0.20 to 1.40.

8. The method of claim 7, wherein said alkylated cyclodextrin has an MS ranging from 0.30 to 1.30.

9. The method of claim 8, wherein said alkylated cyclodextrin has an MS ranging from 0.40 to 1.20.

10. The method of claim 9, wherein said alkylated cyclodextrin has an MS ranging from 0.50 to 1.10.

11. The method of any of claims 4 to 10, wherein said alkylated cyclodextrin is hydroxypropyl-cyclodextrin or methyl-cyclodextrin.

12. A therapeutic cell composition comprising a cyclodextrin compound as defined in anyone of claims 1 to 11.

13. The therapeutic cell composition of claim 12 wherein said therapeutic cells are CAR T-cells.
